(19) Europäisches Patentamt
European Patent Office
Office européen des brevets

(11) EP 0 623 342 B1

# (12) FASCICULE DE BREVET EUROPEEN

(45) Date de publication et mention de la délivrance du brevet: **17.09.1997 Bulletin 1997/38**

(51) Int. Cl.[6]: **A61K 7/48**

(21) Numéro de dépôt: **94401003.2**

(22) Date de dépôt: **06.05.1994**

(54) **Utilisation d'un alkyle ester de glutathion dans une composition cosmétique ou dermatologique destinée au traitement par voie topique du vieillissement cutané**

Verwendung von einem Alkylester in einer kosmetischen oder dermatologischen Zusammensetzung zur topischen Behandlung von der Hautalterung

Use of an alkyl ester of glutathione in a cosmetic or dermatological composition for the topical treatment of skin ageing

(84) Etats contractants désignés:
**CH DE ES FR GB IT LI**

(30) Priorité: **07.05.1993 FR 9305513**

(43) Date de publication de la demande:
**09.11.1994 Bulletin 1994/45**

(73) Titulaire: **L'OREAL**
**75008 Paris (FR)**

(72) Inventeurs:
- **N'Guyen, Quang Lan**
  **F-92160 Antony (FR)**
- **Junino, Alex**
  **F-93190 Livry-Gargan (FR)**
- **Colin, Christian**
  **F-75020 Paris (FR)**
- **Lindenbaum, Albert**
  **F-78000 Versailles (FR)**
- **Loufrani, Catherine**
  **F-94170 Le Perreux sur Marne (FR)**

(74) Mandataire: **Stalla-Bourdillon, Bernard**
**CABINET NONY & CIE**
**29, rue Cambacérès**
**75008 Paris (FR)**

(56) Documents cités:
**WO-A-92/19224** **FR-A- 1 092 020**

- **PATENT ABSTRACTS OF JAPAN vol. 17, no. 391 (C-1087)22 Juillet 1993 & JP-A-05 070 333 (KOSE CORP.) 23 Mars 1993**
- **CHEMICAL ABSTRACTS, vol. 103, no. 9, 2 Septembre 1985, Columbus, Ohio, US; abstract no. 67541r, M.E. ANDERSON 'glutathione monoethyl ester: preparation, uptake by tissues, and conversion to glutathione'**
- **DATABASE WPI Week 9138, Derwent Publications Ltd., London, GB; AN 91-278286 & JP-A-3 184 922 (POLA CHEM IND KK) 14 Décembre 1989**
- **CHEMICAL ABSTRACTS, vol. 102, no. 2, 14 Janvier 1985, Columbus, Ohio, US; abstract no. 12207k, 'ointments inhibiting melanin formation'**

EP 0 623 342 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet l'utilisation d'un alkyle ester de glutathion comme agent protecteur des enzymes cutanées dans la préparation d'une composition cosmétique ou dermatologique destinée au traitement par voie topique du vieillissement cutané.

On sait que les mécanismes de protection de la peau, notamment contre les stress oxydatifs du type ultra-violet font intervenir un certain nombre d'enzymes cutanées de détoxification tels que les catalases et les glutathion peroxydases. Bien que les mécanismes soient particulièrement complexes et qu'ils ne soient que partiellement élucidés, ils font intervenir de façon bien connue les réactions suivantes :

$$2H_2O_2 \xrightarrow[\text{catalase}]{} 2H_2O + O_2 \qquad (1)$$

$$ROOH + 2GSH \xrightarrow[\text{catalase}]{} ROH + GSSG + H_2O \qquad (2)$$

ROOH = peroxyde d'hydrogène ou organique
GSH = glutathion réduit
GSSG = glutathion oxydé
ROH = eau ou alcool
Gpx = - glutathion peroxydase Seleno dépendante lorsque le substrat est le peroxyde d'hydrogène ou un peroxyde organique,
- glutathion transferase lorsque le substrat est un peroxyde organique, ces enzymes étant regroupées sous le terme générique de glutathion peroxydase.

Les catalases et glutathion peroxydases, par leurs interventions dans le processus anti-radicalaire, jouent donc un rôle essentiel dans les mécanismes de protection de la peau mais leur activité est affectée par un stress oxydatif prolongé.

Afin de restaurer l'activité catalasique totale, une addition de catalase exogène serait probablement efficace, mais est malheureusement prohibée en cosmétique.

Par ailleurs, il a été décrit dans le brevet US n° 4.879.370, l'utilisation d'alkyles esters de glutathion comme agents permettant d'augmenter le taux intracellulaire de glutathion après traitement par un agent inhibiteur de la synthèse du glutathion endogène, ainsi que leur éventuelle utilisation comme agent de protection cellulaire.

Toutefois, ce document ne décrit que des utilisations par voie intrapéritonéale des alkyles esters de glutathion à des fins thérapeutiques.

Après de nombreuses études, on a maintenant constaté de façon surprenante et inattendue que par l'utilisation d'un alkyle ester de glutathion en application topique, on pouvait limiter le vieillissement cutané, en agissant conjointement sur les différentes enzymes intervenant dans les mécanismes de détoxification.

La présente invention a donc pour objet l'utilisation d'un alkyle ester de glutathion correspondant à la formule suivante :

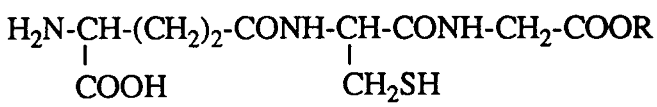

dans laquelle :

R représente un radical alkyle ayant de 1 à 10 atomes de carbone, pour la préparation d'une composition cosmétique ou dermatologique destinée au traitement par voie topique du vieillissement cutané.

Par l'expression "cutané", on doit entendre non seulement la peau mais également le cuir chevelu ainsi que les muqueuses.

Selon l'invention, l'alkyle ester de glutathion utilisé est de préférence le monométhylester, le monoéthylester ou le monooctylester de glutathion.

Les monoalkylesters de glutathion peuvent être préparés selon des méthodes connues notamment selon le procédé décrit dans le brevet US n° 4.879.370 ou bien par voie biotechnologique.

L'alkyle ester de glutathion est utilisé selon l'invention dans la préparation de ladite composition à une concentration comprise entre 0,01 % et 10 %, et de préférence entre 0,5 et 5% en poids par rapport au poids total de la composition.

Selon une forme particulière de réalisation de l'invention, l'alkyle ester de glutathion est utilisé en association avec un principe actif additionnel, présent dans la composition à une concentration comprise entre 0,001 et 10 % en poids par rapport au poids total de la composition.

Ce principe actif additionnel peut être choisi parmi tous les principes actifs utilisés couramment en cosmétologie et en dermatologie, en particulier ceux ayant une activité de dismutation de l'anion superoxyde tel que par exemple la superoxyde dismutase (SOD).

Par l'expression "SOD" on entend tout composant à activité superoxyde-dismutase à savoir tout enzyme qui peut catalyser une réaction de dismutation ainsi que tout autre produit ayant cette activité ce qui inclut notamment les SOD modifiées par greffage de polyalkylène oxyde, polyéthylèneglycol, polysaccharide ou groupes acylés ainsi que les substances contenant de tels produits. On peut citer à cet égard la demande de brevet européen n° EP 223.257.

La SOD utilisable selon l'invention peut ainsi être celle modifiée notamment selon l'enseignement donné dans "International Conference on Medical, Biochemical and Chemical Aspects of Free Radicals" (Avril 9-13, 1988 Kyoto) p.317 de l'article de H. Morimoto, ou selon l'enseignement de M. Yukio Ando p.318 (même source), ou encore d'après les demandes de brevet japonais JP 01250304 et JP 02273176, les demandes de brevet européen EP 426 488 et 424 033 et le brevet US 5.006.333.

La SOD utilisable selon l'invention peut être en outre employée sous forme stabilisée à l'aide de techniques connues par exemple au phosphate, en présence de chlorure de métal alcalin et de saccharose tels que publiées par exemple dans le brevet français FR 2.634.125.

Parmi les substances ayant une activité de type SOD, on peut également citer le 3',5'diisopropyl salicylate de cuivre dont l'utilisation a été décrite dans la demande de brevet européen EP 293.579.

Toutes les superoxyde-dismutases ci-dessus décrites ainsi que les variantes et équivalentes que l'homme du métier peut déduire de cette littérature conviennent en tant que SOD utilisables selon l'invention. Elles peuvent être d'origines diverses.

On peut notamment citer les SOD d'origine animale (par exemple bovine, porcine,...), humaine (par exemple placenta, sang, ....), végétale (par exemple champignons, algues, épinards,.....). Elles peuvent également être obtenues à partir de bactéries ou de levures, ou bien encore par voie biotechnologique (par exemple manipulation génétique,...).

Parmi les exemples de SOD d'origine bovine, on peut citer en particulier la SOD de type Cu-Zn qui a été purifiée jusqu'à homogénéité et approuvée pour des applications cliniques (New Trends in Allergy, I. Ring et al., Ed. Springer Verlag 1986).

Parmi les exemples de SOD obtenue par voie biotechnologique à partir notamment de cultures de bactéries, levures, cellules animales, etc., on peut citer la SOD Cu-Zn humaine recombinante de la Société UBE Industries Ltd.

Parmi les exemples de SOD extraites de bactéries, ont peut citer en particulier celles extraites d'Escherichia coli ; parmi les superoxyde-dismutases extraites de champignons, on peut citer en particulier celles extraites de Pleurotus olearius ; parmi les superoxyde-dismutases extraites du sang, on peut citer les érythrocupréines.

On peut citer également les superoxyde-dismutases extraites de souches bactériennes marines, telles que par exemple des souches de Photobacterium phosphoreum, Photobacterium leiognathi ou Photobacterium sepia.

Parmi les diverses souches utilisables, on peut citer les souches de Photobacterium phosphoreum n° ATCC 11040, de Photobacterium leiognathi n° ATCC 25521, de Photobacterium sepia n° ATCC 15709, d'Escherichia coli n° ATCC 15224 et de Pleurotus olearius Gillet (Laboratoire de Cryptogamie de Paris).

Les SOD utilisables selon l'invention peuvent également être préparées par application des méthodes déjà décrites par exemple dans l'article de Keele et al. (J. Biol. Chem., 245 p. 6176, 1970) ainsi que dans Eur. J. Rheumatol. and Inflammation 4, 173-182 (1982).

Les valeurs d'activité enzymatique SOD de la présente demande ont toutes été exprimées en unités selon Mac Cord et Fridovitch [J. Biol. Chem. 244, 6049 (1969)].

Ainsi, il a pu être déterminé, quelque soit l'origine de la SOD, que la quantité préférée suffisante de SOD pour 100 g de composition est celle qui correspond à une activité d'environ 30 unités à environ 1000 unités dans la composition. Mais de manière générale, la SOD peut posséder de 10 unités à 5.000 unités pour 100 g de composition.

Les compositions selon l'invention peuvent également comprendre, en association avec les alkyle esters de glutathion, des composés choisis parmi les chélatants de métaux et en particulier du fer libre tels que les dérivés phosphoniques décrits dans la demande de brevet français FR 91.05464 (Publication n° 2.675.997), les dérivés de l'EDTA, du DTPA, le desferal, la lactoferrine, la transferrine, la ferritine ou l'acide phytique.

Les compositions à base d'un alkyle ester de glutathion utilisées dans le traitement du vieillissement cutané se présentent sous la forme de solutions aqueuses telles que des sérums, des lotions, des gels, des pommades, des émulsions du type crème ou lait, des pâtes ainsi que des systèmes à base de dispersions vésiculaires ou de dispersions de nanocapsules telles que décrites dans la demande de brevet français n° 90.03418 (Publication n° 2.659.554).

Sous cette dernière forme, les vésicules contiennent par exemple au moins un ingrédient actif tel que décrit ci-dessus, incorporé dans des micelles ou des bicouches lipidiques, encapsulant une phase aqueuse.

Les compositions selon l'invention peuvent également se présenter sous forme de poudre, ou de bâtonnets solides

ou être conditionnées dans des flacons pressurisés et être appliquées sous forme de mousses ou de sprays.

Ces compositions à base d'un alkyle ester de glutathion utilisées dans le traitement du vieillissement cutané peuvent comprendre en outre des ingrédients et des adjuvants utilisés habituellement dans la réalisation des compositions cosmétiques ou dermatologiques tels que des silicones, des agents épaississants, des agents tensioactifs, des polymères, des corps gras solides comme par exemples des cires ou de la lanoline, des agents séquestrants, des agents colorants, des parfums, des substances absorbant les ultra-violets, des agents autobronzants comme de la dihydroxyacétone ainsi que des charges solides comme des poudres ou des pigments.

Parmi les pigments utilisables selon l'invention, on peut citer la mélanine qu'elle soit d'origine naturelle par exemple issue des cheveux ou d'encre de céphalopodes ou d'origine synthétique, les pigments résultant de polymérisation oxydante ou enzymatique de précurseurs comme la L-tyrosine, la L-dopa, le catéchol et leurs dérivés, ou bien encore des pigments d'oxydes métalliques tels que les oxydes de titane, de zinc, de cérium ou de zirconium.

Lorsque les compositions selon l'invention comprennent lesdits pigments d'oxydes métalliques, ceux-ci sont présents à une concentration comprise entre 0,1 et 15 % et de préférence entre 0,5 et 10 % en poids par rapport au poids total de la composition.

Ces pigments sont utilisés de préférence sous la forme de nanopigments, de diamètre moyen inférieur à 100 nm, et en particulier compris entre 5 et 50 nm.

Ces pigments peuvent être également enrobés par des composés tels que des acides aminés, des tensioactifs anioniques, des lécithines, des alcools gras, de la cire d'abeille, des acides gras, des sels d'acides gras notamment de sodium, de potassium, de zinc, de fer ou d'aluminium, des alkoxydes métalliques notamment de titane ou d'aluminium, du polyéthylène, des silicones, des protéines notamment du collagène ou de l'élastine, des alcanolamines, des oxydes de silicium, des oxydes métalliques ou de l'hexamétaphosphate de sodium.

Les compositions anti-vieillissement selon l'invention sont utilisables non seulement pour les soins de la peau ainsi que pour sa protection, mais trouvent également une application directe dans les produits solaires et dans les produits de maquillage.

Les compositions anti-vieillissement selon l'invention sont utilisables également sur le cuir chevelu pour le ralentissement de la chute des cheveux et pour favoriser la repousse des cheveux. Ces compositions peuvent contenir en association certains composés actifs tels que le "Minoxidil" (2,4-diamino-6-piperidino-pyrimidine-3-oxyde) et ses dérivés, le diazoxide (7-choro-3-méthyl-1,2,4-benzothiadiazine 1,1-dioxyde) et le "Phenytoïn" (5,5-diphényl imidazolidine 2,4-dione).

Les compositions selon l'invention peuvent en outre trouver une utilisation bucco-dentaire en particulier sous forme de pâtes dentifrices. Dans ce cas, la composition comprend des adjuvants et additifs usuels pour les compositions à usage buccal et notamment des agents tensio-actifs, des agents épaississants, des agents humectants et des agents de polissage tels que la silice, des agents actifs comme les fluorures, et en particulier le fluorure de sodium et éventuellement des agents édulcorants comme le saccharinate de sodium.

On va maintenant donner à titre d'illustration plusieurs exemples de compositions selon l'invention.

**EXEMPLE 1 -** *Crème anti-vieillissement pour le visage*

| *A. Phase grasse* | |
|---|---|
| - Monostéarate de polyéthylèneglycol 50 OE (ICI) | 1,5 % |
| - Mélange de mono et distéarate de diglycérol | 1,5 % |
| - Huile de vaseline | 24 % |
| - Alcool cétylique | 2,5 % |

| *B. Phase aqueuse* | |
|---|---|
| - Monoéthylester de glutathion | 0,5 % |
| - Eau q.s.p. | 100 % |

(Dans cet exemple ainsi que dans les exemples suivants, les pourcentages sont exprimés en poids par rapport au poids de la composition finale).

On prépare la crème de la manière suivante :

On chauffe la phase grasse A à 80°C, puis on y verse, sous vive agitation, la phase aqueuse B.

La crème obtenue présente un très bel aspect et possède de bonnes propriétés cosmétiques lorsqu'elle est appliquée sur le visage.

**EXEMPLE 2 :** *Fluide de soin anti-vieillissement pour le corps*

On prépare d'abord, de façon connue, un support sous la forme d'une dispersion de sphérules lipidiques, de composition suivante (en % par rapport à la composition finale) :

- Lipide amphiphile non ionique de formule $R\text{-}(O\text{-}\underset{\displaystyle CH_2OH}{\underset{|}{C}H}\text{-}CH_2)_n\text{-}OH$ dans laquelle R est un radical hexadécyle et n a une valeur statistique moyenne égale à 3 — 4,5 %
- Cholestérol — 4,5 %
- Dicétylphosphate — 1,0 %
- Parahydroxybenzoate de méthyle — 0,3 %
- Eau déminéralisée stérile — 30 %

Pour cela, on mélange par fusion à 100°C, les trois premiers ingrédients, sous atmosphère d'azote, on refroidit à 80°C puis homogénéise à l'aide d'un ultradisperseur de type Virtis. On ajoute ensuite l'eau et l'agent conservateur. Après avoir ramené la dispersion à la température ambiante, on y ajoute alors 1 % de monoéthylester de glutathion puis la phase A suivante :

| *Phase A :* | |
|---|---|
| - Parfum | 0,4 % |
| - Huile de tournesol | 10 % |
| - Huile de paraffine | 4 % |
| - Vitamine F | 2 % |
| - Lécithine de soja | 1 % |
| - Palmitate d'ascorbyle | 1 % |
| - Salicylate d'hexadécylamine | 0,2 % |

On homogénéise le mélange à l'aide d'un ultradisperseur et on disperse ensuite la phase B suivante :

| *Phase B :* | |
|---|---|
| - Acide polyacrylique réticulé commercialisé sous la dénomination "Carbopol® 940" par la Société Goodrich | 0,4 % |
| - Eau déminéralisée | 39,3 % |

L'ensemble est enfin neutralisé à l'aide de 0,4 % de triéthanolamine.

Le fluide de soin ainsi obtenu peut être utilisé par application sur le corps pour apporter de la douceur à la peau ainsi qu'une amélioration de son aspect.

**EXEMPLE 3 :** *Lait de beauté protecteur pour le corps*

Ce lait se présente sous forme d'une émulsion huile-dans-l'eau (H/E) ayant la composition suivante :

| | |
|---|---|
| - Monoéthylester de glutathion | 1 % |
| - Huile de purcellin (Dragoco) | 2 % |
| - Huile de vaseline | 6 % |
| - Alcool oléique | 1% |
| - Myristate d'isopropyle | 1,5 % |
| - Monostéarate de glycérine | 2 % |
| - Stéarine | 1,4 % |
| - Alcool cétylique | 0,1 % |
| - Parfum | 0,9 % |
| - Acide polyacrylique réticulé commercialisé sous la dénomination "Carbopol® 941" par la Société Goodrich | 0,35 % |
| - Triéthanolamine pure | 1,05 % |
| - Parahydroxybenzoate de butyle | 0,04 % |
| - Agent conservateur | 0,3 % |
| - Propylèneglycol | 5% |
| - Mélange hydratant (lactate de sodium/TEA lactate/sérine/urée/acide lactique) commercialisé sous la dénomination "Hydroviton" par la Société Dragoco | 1,5 % |
| - Colorant F.D.C. blue 1 (Kohnstamn) à 1 % dans l'eau | 0,03 % |
| - Eau déminéralisée q.s.p. | 100 % |

**EXEMPLE 4 :** *Crème anti-vieillissement pour le corps*

Cette crème se présente sous forme d'une émulsion H/E ayant la composition suivante :

| | |
|---|---|
| - Monoéthylester de glutathion | 0,8 % |
| - Alcool cétylique | 0,5 % |
| - Cire de sipol | 5 % |
| - Monostéarate de glycérol | 1,5 % |
| - Huile de vaseline | 6 % |
| - Myristate d'isopropyle | 3 % |
| - Glycérine | 10 % |
| - Parfum | 0,2 % |
| - Eau q.s.p. | 100 % |

**EXEMPLE 5 :** *Crème anti-vieillissement pour le corps*

Cette crème se présente sous forme d'une émulsion H/E ayant la composition suivante :

| | |
|---|---|
| - Monoéthylester de glutathion | 0,5 % |
| - Cire de Sipol | 6 % |
| - Monostéarate de glycérol | 1,5 % |
| - Stéarate de sodium | 0,8 % |
| - Huile de vaseline | 6 % |
| - Palmitate d'isopropyle | 2 % |
| - Glycérine | 15 % |
| - Parfum | 0,3 % |
| - Eau q.s.p. | 100 % |

**EXEMPLE 6 :** *Gel dermatopharmaceutique anti-rides*

On mélange, à température ambiante et sous agitation, les composés suivants :

| | |
|---|---|
| - Monoéthylester de glutathion | 0,6 % |
| - Polymère séquencé de polyoxyéthylène/polyoxypropylène/polyoxyéthylène "Poloxamer 182" commercialisé sous la dénomination "Synperonic PE/L62" par la Société ICI | 0,2 % |
| - Propylène glycol | 4 % |
| - Acide lactique | 1 % |
| - Sel tétrasodique de l'acide éthylènediamine tétracétique | 0,1 % |
| - Phénoxyéthanol | 0,25 % |
| - Eau q.s.p. | 100 % |

On ajoute à la dispersion obtenue 1 % de "Carbopol 940", puis de la soude en quantité suffisante pour ajuster le pH à 5.

Le gel obtenu est appliqué sur le visage et le cou.

**EXEMPLE 7 :** *Crème de soin protectrice anti-vieillissement*

| | |
|---|---|
| - Monoéthylester de glutathion | 0,7 % |
| - Mélange d'alcool cétylstéarylique et d'alcool cétylstéarylique oxyéthyléné à 33 OE commercialisé sous la dénomination de "Sinnowax AO" par la Société Henkel | 5 % |
| - Stéarate de glycérol | 1 % |
| - Alcool cétylique | 1% |
| - Huile de jojoba | 6 % |
| - Acide linoléique | 6% |
| - Oxyde de titane commercialisé sous la dénomination "MT 100 T" par la Société Tayca | 5 % |
| - Conservateur q.s. | |
| - Eau q.s.p. | 100 % |

On chauffe la phase grasse à 80°C, puis on ajoute l'oxyde de titane. On verse alors la phase aqueuse, sous agitation à 80°C, dans la phase grasse.

Cette composition sous forme de crème est appliquée quotidiennement sur le visage pour retarder l'apparition des rides.

**EXEMPLE 8 :** *Sérum antichute pour le cuir chevelu*

| | |
|---|---|
| - Monoéthylester de glutathion | 0,20 % |
| - Polyglycéryl-2 stéarate commercialisé sous la dénomination de "Hostacerine DGS" par la Société Hoechst | 1,30 % |
| - Cholestérol | 0,60 % |
| - Sel de sodium de l'acide phosphatidique | 0,10 % |
| - Glycérine | 3,00 % |
| - L-hydroxyproline | 1,00 % |
| - D-panthénol | 1,50 % |
| - Guanosine | 0,01 % |
| - Conservateurs | 0,30 % |
| - Polyphosphonate commercialisé sous la dénomination de "Dequest 2046" par la Société Monsanto Chemical | 0,80 % |
| - Hydrolysat lactique commercialisé sous la dénomination de "Lactolan LS" par les laboratoires sérobiologiques de Nancy | 5,00 % |
| - Minoxidil | 1,00 % |
| - Céruloplasmine | 0,01% |
| - Solution aqueuse de superoxyde dismutase à 5000 unités/ml commercialisée par la Société Pentapharm | 1,00 % |
| - Mélange d'acides polycarboxyvinyliques commercialisé sous la dénomination de "Carbopol® 940" par la Société Goodrich | 0,50 % |
| - Triéthanolamine q.s. pH 6,5 | |
| - Eau déminéralisée q.s.p. | 100 % |

**EXEMPLE 9 :** *Crème anti-vieillissement dépigmentante à base de liposomes*

***a)*** On prépare d'abord une dispersion de vésicules lipidiques.

Pour cela, on mélange par fusion à 110°C sous atmosphère d'azote, les ingrédients suivants :

| | |
|---|---|
| - Lipide amphiphile non ionique de formule $R-(O-\underset{\displaystyle CH_2OH}{\underset{\vert}{CH}}-CH_2)_n-OH$ dans laquelle R est un radical hexadécyle et n a une valeur statistique moyenne égale à 3 | 3,75 % |
| - Cholestérol | 3,75 % |

Aprés refroidissement du mélange à 90°C, on ajoute :

| | |
|---|---|
| - Acide n-heptanoyl-5-salicylique | 0,50 % |

Après homogénéisation à l'aide d'un ultradisperseur du type Virtis, on incorpore les ingrédients suivants :

| | |
|---|---|
| - Glycérine | 3,00 % |
| - Eau déminéralisée | 19,30 % |

On refroidit le mélange à 70°C, puis homogénéise et ajoute :

| | |
|---|---|
| - Monoéthylester de glutathion | 0,60 % |
| - Acide kojique | 1,00 % |
| - Eau déminéralisée | 22,42 % |

On abaisse la température du mélange à 40°C, l'homogénéise et y additionne enfin les produits suivants :

| | |
|---|---|
| - Acide caféique | 1,27 % |
| - Monoéthyléther de diéthylèneglycol | 7,50 % |

Après homogénéisation, la dispersion obtenue présente des vésicules lipidiques dont la taille moyenne est d'environ 0,2 microns.

***b)*** On prépare ensuite la crème.

Pour cela, on ajoute à 25°C à la dispersion précédemment obtenue, les ingrédients suivants :

| | |
|---|---|
| - Huile de vaseline | 14,00 % |
| - Huile de silicone volatile | 10,00 % |

Après homogénéisation à l'aide d'un ultradisperseur, on incorpore les produits suivants :

| | |
|---|---|
| - Parfum | 0,40 % |
| - Mélange d'acides polycarboxyvinyliques commercialisé sous la dénomination "Carbopol® 940" par la Société Goodrich | 0,40 % |
| - Triéthanolamine | 1,50 % |
| - Parahydroxybenzoate de méthyle | 0,20 % |
| - Eau déminéralisée q.s.p. | 100 % |

La crème ainsi obtenue est de couleur beige et possède de bonnes propriétés cosmétiques.

**EXEMPLE 10 :** *Gel anti-vieillissement et anti-chute pour le cuir chevelu*

| | |
|---|---|
| - Monoéthylester de gluthation | 0,20 % |
| - Superoxyde dismutase | 30 UI |
| - Polyphosphonate commercialisé sous la dénomination de "Dequest 2046" par la Société Monsanto Chemical | 0,07 %MA |
| - Propylèneglycol | 5 % |
| - Ceruloplasmine | 0,01 % |
| - Acide polyacrylique réticulé commercialisé sous la dénomination "Carbopol® 941" par la Société Goodrich | 0,50 % |
| - Minoxidil | 1,00 % |
| - Triéthanolamine q.s. pH 7 | |
| - Conservateurs q.s. | |
| - Eau q.s.p. | 100 % |

**EXEMPLE 11 :** *Crème de soin pour le visage sous forme d'une émulsion huile-dans-l'eau*

| | |
|---|---|
| - Monoéthylester de glutathion | 0,50 % |
| - Superoxyde dismutase titrant à 3,13 U/mg q.s.p. 600 unités commercialisée par la Société Pentapharm | 0,20 % |
| - Polyéthylèneglycol 50 oxyéthyléné | 1,50 % |
| - Stéarate de monodiglycéryle | 1,50 % |
| - Huile de vaseline | 24,00 % |
| - Alcool cétylique | 2,50 % |
| - Triéthanolamine q.s. pH 7 | |
| - Eau q.s.p. | 100 % |

On prépare une crème de la manière suivante.

On chauffe le mélange des ingrédients à l'exception de la SOD à 80°C, puis après homogénéisation et refroidissement du mélange ainsi obtenu à 30°C, on ajoute la SOD.

**EXEMPLE 12 :** *Emulsion eau-dans-l'huile*

| | |
|---|---|
| - Mono-octylester de glutathion | 0,5 % |
| - Polyéthylène glycol 40 | 1,5 % |
| - Stéarate de monodiglycéryle | 1,5 % |
| - Huile de vaseline | 24 % |
| - Alcool cétylique | 2,5 % |
| - Tristéarate de sorbitan | 0,3 % |
| - Eau q.s.p. | 100 % |

**EXEMPLE 13 :** *Emulsion huile-dans-l'eau*

| | |
|---|---|
| - Monométhylester de glutathion | 0,5 % |
| - Polyéthylène glycol 40 | 1,5 % |
| - Stéarate de monodiglycéryle | 1,5 % |
| - Huile de vaseline | 24 % |
| - Alcool cétylique | 2,5 % |
| - Stéarate de glycéryle | 1 % |
| - Eau q.s.p. | 100 % |

**EXEMPLE 14 :** *Emulsion eau-dans-l'huile*

| | |
|---|---|
| - Monométhylester de glutathion | 1 % |
| - Cétyl diméthicone copolyol | 3 % |
| - Gel de bentone | 5 % |
| - Silicone volatil | 10 % |
| - Huile de beurre de karité | 5 % |
| - Purcellin liquide | 3 % |
| - Sulfate de magnésium | 0,7 % |
| - Glycérol | 3 % |
| - Eau q.s.p. | 100 % |

**Revendications**

1. Utilisation d'un alkyle ester de glutathion correspondant à la formule :

$$H_2N\text{-}\underset{\displaystyle COOH}{\underset{|}{CH}}\text{-}(CH_2)_2\text{-CONH-}\underset{\displaystyle CH_2SH}{\underset{|}{CH}}\text{-CONH-}CH_2\text{-COOR} \qquad (I)$$

   dans laquelle :

   R représente un radical alkyle ayant de 1 à 10 atomes de carbone, pour la préparation d'une composition cosmétique ou dermatologique destinée au traitement par voie topique du vieillissement cutané.

2. Utilisation selon la revendication 1, caractérisée par le fait que ledit alkyle ester de glutathion est le monométhylester, le monoéthylester ou le monooctylester de glutathion.

3. Utilisation selon la revendication 1 ou 2, caractérisée par le fait que ledit alkyle ester de glutathion est présent dans la composition à une concentration comprise entre 0,01 % et 10 %, et de préférence entre 0,5 et 5 % en poids par rapport au poids total de la composition.

4. Utilisation selon l'une quelconque des revendications précédentes, caractérisée par le fait que ladite composition comprend en outre un principe actif additionnel à une concentration comprise entre 0,001 et 10 % en poids par rapport au poids total de la composition.

5. Utilisation selon la revendication 4, caractérisée par le fait que ledit principe actif additionnel est choisi parmi la superoxyde dismutase purifiée ou non, éventuellement modifiée ou une substance ayant une activité de superoxyde dismutase.

6. Utilisation selon l'une quelconque des revendications précédentes, caractérisée par le fait que ladite composition comprend en outre au moins un agent de chélation des métaux.

7. Utilisation selon l'une quelconque des revendications précédentes, caractérisée par le fait que ladite composition comprend en outre des pigments d'oxydes métalliques.

**8.** Utilisation selon la revendication 7, caractérisée par le fait que lesdits pigments sont présents dans la composition à une concentration comprise entre 0,1 et 15 % et de préférence entre 0,5 et 10 % en poids par rapport au poids total de la composition.

**9.** Composition cosmétique ou dermatologique destinée au traitement par voie topique du vieillissement cutané, caractérisée par le fait qu'elle contient un alkyle ester de glutathion de formule (I) et une superoxyde dismutase.

**10.** Composition cosmétique ou dermatologique selon la revendication 9, caractérisée par le fait que ledit alkyle ester de glutathion est présent en une proportion comprise entre 0,01 % et 10 % et de préférence entre 0,5 et 5 % et que ladite superoxyde dismutase est présente en une proportion comprise entre 0,001 et 10 % en poids par rapport au poids total de la composition.

**Claims**

**1.** Use of a glutathione alkyl ester corresponding to the formula:

$$\begin{array}{l} H_2N\text{-}\underset{\displaystyle COOH}{\underset{|}{C}H}\text{-}(CH_2)_2\text{-}CONH\text{-}\underset{\displaystyle CH_2SH}{\underset{|}{C}H}\text{-}CONH\text{-}CH_2\text{-}COOR \end{array} \qquad (I)$$

in which:

R represents an alkyl radical having from 1 to 10 carbon atoms, for the preparation of a cosmetic or dermatological composition intended for the topical treatment of cutaneous ageing.

**2.** Use according to Claim 1, characterized by the fact that the said glutathione alkyl ester is glutathione monomethyl ester, monoethyl ester or monooctyl ester.

**3.** Use according to Claim 1 or 2, characterized by the fact that the said glutathione alkyl ester is present in the composition at a concentration of between 0.01 % and 10 %, and preferably between 0.5 and 5 % by weight relative to the total weight of the composition.

**4.** Use according to any one of the preceding claims, characterized by the fact that the said composition comprises, in addition, an additional active ingredient at a concentration of between 0.001 and 10 % by weight relative to the total weight of the composition.

**5.** Use according to Claim 4, characterized by the fact that the said additional active ingredient is chosen from optionally modified purified or unpurified superoxide dismutase or a substance having a superoxide dismutase activity.

**6.** Use according to any one of the preceding claims, characterized by the fact that the said composition comprises, in addition, at least one metal-chelating agent.

**7.** Use according to any one of the preceding claims, characterized by the fact that the said composition comprises, in addition, pigments of metallic oxides.

**8.** Use according to Claim 7, characterized by the fact that the said pigments are present in the composition at a concentration of between 0.1 and 15 % and preferably between 0.5 and 10 % by weight relative to the total weight of the composition.

**9.** Cosmetic or dermatological composition intended for the topical treatment of cutaneous ageing, characterized by the fact that it contains a glutathione alkyl ester of formula (I) and a superoxide dismutase.

**10.** Cosmetic or dermatological composition according to Claim 9, characterized by the fact that the said glutathione alkyl ester is present in a proportion of between 0.01 % and 10 % and preferably between 0.5 and 5 % and that the said superoxide dismutase is present in a proportion of between 0.001 and 10 % by weight relative to the total weight of the composition.

## Patentansprüche

1. Verwendung eines Glutathionalkylesters, der der folgenden Formel entspricht:

$$H_2N-\underset{\displaystyle COOH}{\underset{|}{CH}}-(CH_2)_2-CONH-\underset{\displaystyle CH_2SH}{\underset{|}{CH}}-CONH-CH_2-COOR \qquad (I)$$

worin

R einen Alkylrest mit 1 bis 10 Kohlenstoffatomen bedeutet, zur Herstellung einer kosmetischen oder dermatologischen Zubereitung, die zur Behandlung der Hautalterung auf topischem Wege bestimmt ist.

2. Verwendung nach Anspruch 1, dadurch gekennzeichnet, daß der Glutathionalkylester als Glutathionmonomethylester, Glutathionmonoethylester oder Glutathionmonooctylester vorliegt.

3. Verwendung nach Anspruch 1 oder 2, dadurch gekennzeichnet, daß der Glutathionalkylester in der Zubereitung in einer Konzentration im Bereich zwischen 0,01 und 10 Gew.-%, vorzugsweise zwischen 0,5 und 5 Gew.-% in bezug auf das Gesamtgewicht der Zusammensetzung vorhanden ist.

4. Verwendung nach einem der vorstehenden Ansprüche, dadurch gekennzeichnet, daß die Zubereitung zusätzlich noch einen weiteren Wirkstoff in einer Konzentration im Bereich zwischen 0,001 und 10 Gew.-% in bezug auf das Gesamtgewicht der Zusammensetzung enthält.

5. Verwendung nach Anspruch 4, dadurch gekennzeichnet, daß der zusätzliche weitere Wirkstoff unter gegebenenfalls gereinigter Superoxiddismutase, die gegebenenfalls modifiziert ist, oder einer Substanz mit einer Superoxiddismutasewirkung ausgewählt wird.

6. Verwendung nach einem der vorstehenden Ansprüche, dadurch gekennzeichnet, daß die Zubereitung zusätzlich noch mindestens ein Metallkomplexierungsmittel enthält.

7. Verwendung nach einem der vorstehenden Ansprüche, dadurch gekennzeichnet, daß die Zubereitung zusätzlich noch Metalloxidpigmente enthält.

8. Verwendung nach Anspruch 7, dadurch gekennzeichnet, daß die Pigmente in der Zubereitung in einer Konzentration im Bereich zwischen 0,1 und 15 Gew.-%, vorzugsweise zwischen 0,5 und 10 Gew.-%, in bezug auf das Gesamtgewicht der Zusammensetzung enthalten sind.

9. Kosmetische oder dermatologische Zubereitung, die zur Behandlung der Hautalterung auf dem topischem Verabreichungsweg bestimmt ist, dadurch gekennzeichnet, daß sie einen Glutathionalkylester gemäß der Formel (I) sowie eine Superoxiddismutase enthält.

10. Kosmetische oder dermatologische Zubereitung nach Anspruch 9, dadurch gekennzeichnet, daß der Glutathionalkylester in einem Verhältnis zwischen 0,01 und 10 Gew.-%, vorzugsweise zwischen 0,5 und 5 Gew.-%, und die Superoxiddismutase in einem Mengenverhältnis zwischen 0,001 und 10 Gew.-% in bezug auf das Gesamtgewicht der Zusammensetzung enthalten sind.